# EUROPEAN PATENT SPECIFICATION

(11) **EP 1 305 311 B1**
(45) Date of publication and mention of the grant of the patent: **16.01.2008**
(21) Application number: 01954159.8
(22) Date of filing: 03.08.2001
(51) Int. Cl.: C07D 417/12, A61K 31/44, A61P 3/08

(54) **TARTRATE SALT OF THIAZOLIDINEDIONE DERIVATIVE**
TARTRATSALZE EINES THIAZOLIDINEDIONE DERIVATS
TARTRATE DE DERIVE DE THIAZOLIDINEDIONE

(30) Priority: 04.08.2000 GB 0019224
(43) Date of publication of application: 02.05.2003
(73) Proprietor: SMITHKLINE BEECHAM PLC, Brentford, Middlesex TW8 9GS (GB)
(72) Inventor: CRAIG, Andrew Simon, Tonbridge, Kent TN119AN (GB); HO, Tim Chien Ting, Tonbridge, Kent TN119AN (GB)
(74) Representative: Rutter, Keith
(86) International application number: PCT/GB2001/003511
(87) International publication number: WO 2002/012232

(56) References cited:
- WO-A-94/05659

## Description

This invention relates to a novel pharmaceutical, to a process for the preparation of the pharmaceutical and to the use of the pharmaceutical in medicine.

European Patent Application, Publication Number 0,306,228 relates to certain thiazolidinedione derivatives disclosed as having hypoglycaemic and hypolipidaemic activity. The compound of example 30 of EP 0,306,228 is 5-[4-[2-(N-methyl-N-(2-pyridyl)amino)ethoxy]benzyl]thiazolidine-2,4-dione (hereinafter also referred to as "Compound (I)").

International Patent Application, Publication Number WO94/05659 discloses certain salts of the compounds of EP 0,306,228 one of which is the tartrate salt. The preferred salt of WO94/05659 is the maleic acid salt.

It has now been discovered that Compound (I) forms a novel tartrate salt (hereinafter also referred to as the "DL-Tartrate"). Surprisingly, the crystalline structure of the DL-Tartrate is distinct from that of either the D-tartrate or the L-tartrate salts or solid mixtures of the D-and L-tartrate salts.

The novel DL-Tartrate is a stable, high melting crystalline material hence is suitable for bulk preparation and handling. The DL- Tartrate is amenable to large scale pharmaceutical processing, especially in manufacturing processes which require or generate heat, for example milling, fluid bed drying, spray drying, hot melt processing and sterilisation by autoclaving. The DL-Tartrate can also be prepared by an efficient, economic and reproducible process particularly suited to large-scale preparation.

The novel DL-tartrate also has useful pharmaceutical properties and in particular it is indicated to be useful for the treatment and/or prophylaxis of diabetes mellitus, conditions associated with diabetes mellitus and certain complications thereof.

Accordingly, the present invention provides 5-[4-[2-(N-methyl-N-(2-pyridyl)amino)ethoxy]benzyl]thiazolidine-2,4-dione, DL-Tartrate salt or a solvate thereof.

Suitably, the DL-Tartrate is a mono-tartrate salt.

Mono tartrate salts also optionally comprise another monovalent salting ion such as an alkali metal or ammonium cation.

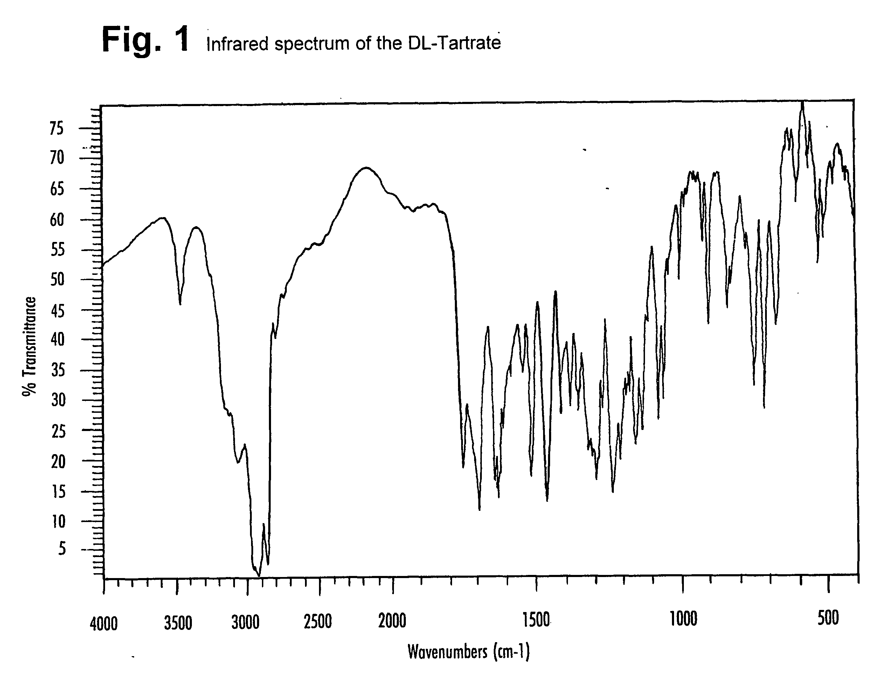

In one favoured aspect, the DL-Tartrate provides an infrared spectrum substantially in accordance with Figure 1.

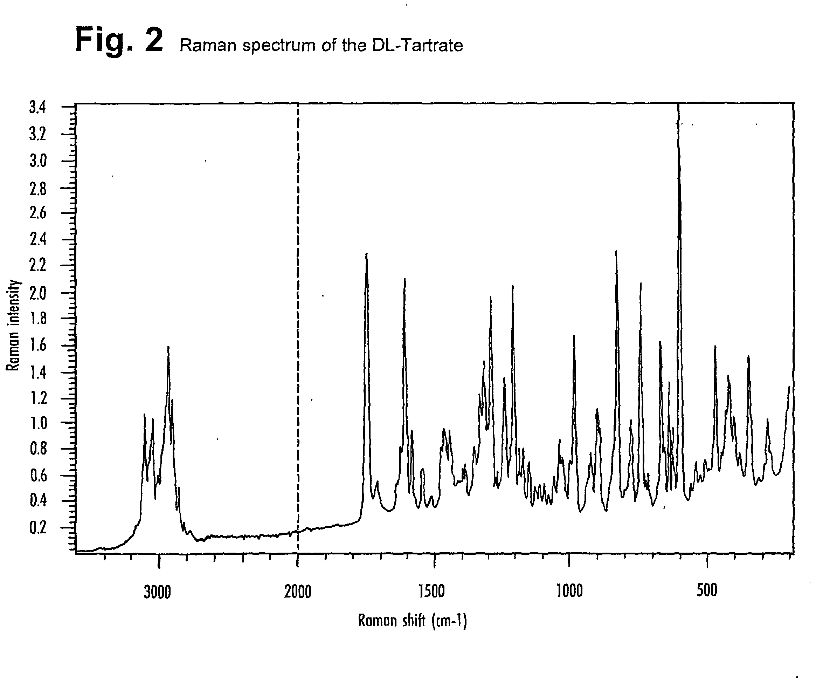

In one favoured aspect, the DL-Tartrate provides a Raman spectrum substantially in accordance with Figure 2.

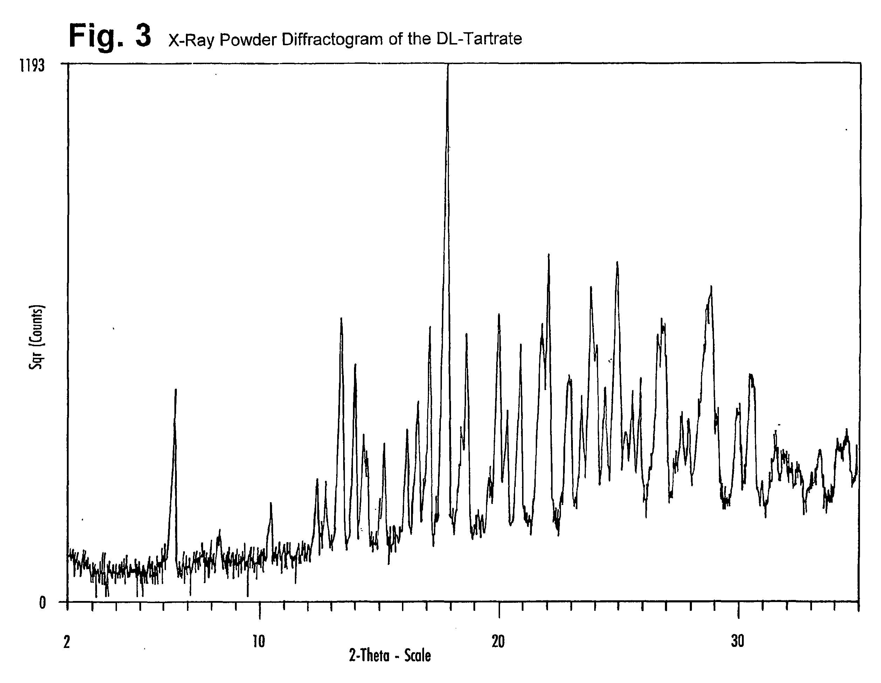

In one favoured aspect, the DL-Tartrate provides an X-Ray powder diffraction pattern (XRPD) substantially in accordance with Table 1 or Figure 3.

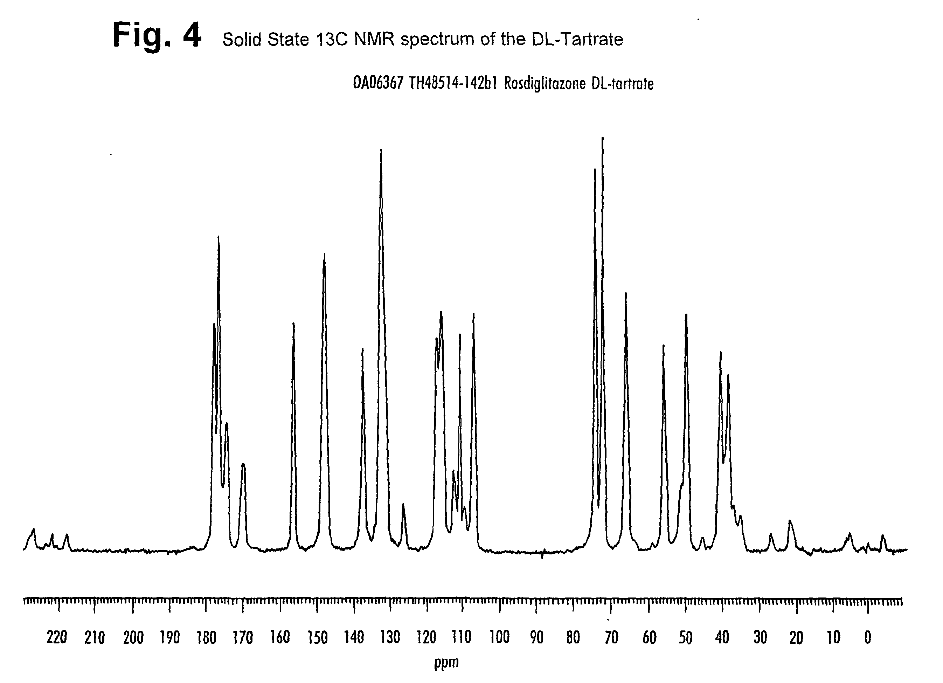

In one favoured aspect, the DL-tartrate provides a Solid State ¹³C NMR spectrum substantially in accordance with Figure 4.

In one favoured aspect, the DL- Tartrate provides a melting point in the range of from 190 to 195°C, such as 190 to 193 °C, for example 191.7 °C

In a preferred aspect, the invention provides 5-[4-[2-(N-methyl-N-(2-pyridyl)amino)ethoxy]benzyl]thiazolidine-2,4-dione, DL-Tartrate salt, characterised in that it provides:
(i) an infrared spectrum substantially in accordance with Figure 1; and
(ii) a Raman spectrum substantially in accordance with Figure 2;and
(iii) an X-Ray powder diffraction pattern (XRPD) substantially in accordance with Table 1 or Figure 3 ;and
(iv) a Solid State ¹³C NMR spectrum substantially in accordance with Figure 4.

The present invention encompasses the DL-Tartrate or solvate thereof isolated in pure form or when admixed with other materials. Thus in one aspect there is provided the DL-Tartrate or solvate thereof in isolated form.

In a further aspect there is provided the DL-Tartrate or solvate thereof in a purified form.

In yet a further aspect there is provided the DL-Tartrate or solvate thereof in crystalline form.

Also, the invention provides the DL-Tartrate or solvate thereof in a solid pharmaceutically acceptable form, such as a solid dosage form, especially when adapted for oral administration.

Moreover, the invention also provides the DL-Tartrate or solvate thereof in a pharmaceutically acceptable form, especially in bulk form, such form being particularly capable of pharmaceutical processing, especially in manufacturing processes which require or generate heat, for example milling; for example heat-drying especially fluid-bed drying or a spray drying; for example hot melt processing; for example heat-sterilisation such as autoclaving.

Furthermore, the invention provides the DL-Tartrate or solvate thereof in a pharmaceutically acceptable form, especially in bulk form and especially in form having been processed in a manufacturing process requiring or generating heat, for example in a milled form; for example in heat-dried form, especially a fluid-bed dried form or a spray dried form; for example in a form having being hot melt processed; for example in a form having being heat-sterilised by such as autoclaving.

A suitable solvate is a hydrate.

The invention also provides a process for preparing the DL-Tartrate or a solvate thereof, characterised in that 5-[4-[2-(N-methyl-N-(2-pyridyl)amino)ethoxy]benzyl]thiazolidine-2,4-dione (Compound (I)) or a salt thereof, preferably dispersed or dissolved in a suitable solvent, is reacted with a source of DL tartrate ion and thereafter, if required, a solvate of the resulting DL-Tartrate is prepared; and the DL-Tartrate or a solvate thereof is recovered.

A suitable reaction solvent is an alkanol, for example propan-2-ol, or a hydrocarbon, such as toluene, a ketone, such as acetone, an ester, such as ethyl acetate, an ether such as tetrahydrofuran, a nitrile such as acetonitrile, or a halogenated hydrocarbon such as dichloromethane or water, or an organic acid such as acetic acid; or a mixture thereof.

Conveniently, the source of DL-tartrate ion is DL-tartaric acid. The DL-tartaric acid is preferably added as a solid or in solution, for example in water or a lower alcohol such as methanol, ethanol, or propan-2-ol, or a mixture of solvents. An alternative source of DL-tartrate ion is provided by a suitably soluble base salt of tartaric acid for example ammonium tartrate, or the tartaric acid salt of an amine, for example ethylamine or diethylamine.

The concentration of Compound (I) is preferably in the range 2 to 25% weight/volume, more preferably in the range 5 to 20%. The concentration of tartaric acid solutions are preferably in the range range of 4 to 40% weight/volume.

The reaction is usually carried out at ambient temperature or at an elevated temperature, for example at the reflux temperature of the solvent, although any convenient temperature that provides the required product may be employed.

Solvates, such as hydrates, of the DL-Tartrate are prepared according to conventional procedures.

Recovery of the required compound generally comprises crystallisation from an appropriate solvent or mixture of solvents, conveniently the reaction solvent, usually assisted by cooling. For example, the DL- tartrate may be crystallised from an alcohol such as ethanol or a ketone such as acetone or water or a mixture thereof. An improved yield of the salt may be obtained by evaporation of some or all of the solvent or by crystallisation at elevated temperature followed by controlled cooling, optionally in stages. Careful control of precipitation temperature may be used to improve the reproducability product form.

Crystallisation can also be initiated by seeding with crystals of the DL-Tartrate or a solvate thereof but this is not essential.

When the mono tartrate salt comprise another monovalent salting ion such as an alkali metal or ammonium cation the said ion is conveniently formed by reacting the mono tartrate salt with a solution of the chosen monovalent salting ion for example a metal or ammonium ion. Alternatively Compound(I) may be treated with a mono tartrate salt of the said monovalent salting ion.

Compound (I) is prepared according to known procedures, such as those disclosed in EP 0,306,228 and WO94/05659. The disclosures of EP 0,306,228 and WO94/05659 are incorporated herein by reference.

DL tartaric acid is a commercially available compound.

When used herein the term "Tₒₙₛₑₜ" is generally determined by Differential Scanning Calorimetry and has a meaning generally understood in the art, as for example expressed in Pharmaceutical Thermal Analysis, Techniques and Applications", Ford and Timmins, 1989 as "The temperature corresponding to the intersection of the pre-transition baseline with the extrapolated leading edge of the transition".

When used herein the term 'prophylaxis of conditions associated with diabetes mellitus' includes the treatment of conditions such as insulin resistance, impaired glucose tolerance, hyperinsulinaemia and gestational diabetes.

Diabetes mellitus preferably means Type II diabetes mellitus.

Conditions associated with diabetes include hyperglycaemia and insulin resistance and obesity. Further conditions associated with diabetes include hypertension, cardiovascular disease, especially atherosclerosis, certain eating disorders, in particular the regulation of appetite and food intake in subjects suffering from disorders associated with under-eating, such as anorexia nervosa, and disorders associated with over-eating, such as obesity and anorexia bulimia. Additional conditions associated with diabetes include polycystic ovarian syndrome and steroid induced insulin resistance.

The complications of conditions associated with diabetes mellitus encompassed herein includes renal disease, especially renal disease associated with the development of Type II diabetes including diabetic nephropathy, glomerulonephritis, glomerular sclerosis, nephrotic syndrome, hypertensive nephrosclerosis and end stage renal disease.

As mentioned above the compound of the invention has useful therapeutic properties: The present invention accordingly provides the DL-Tartrate or a solvate thereof for use as an active therapeutic substance.

More particularly, the present invention provides the DL-Tartrate or a solvate thereof for use in the treatment and/or prophylaxis of diabetes mellitus, conditions associated with diabetes mellitus and certain complications thereof.

The DL-Tartrate or a solvate thereof may be administered per se or, preferably, as a pharmaceutical composition also comprising a pharmaceutically acceptable carrier. Suitable methods for formulating the DL-Tartrate or a solvate thereof are generally those disclosed for Compound (I) in the above mentioned publications.

Accordingly, the present invention also provides a pharmaceutical composition comprising the DL-Tartrate or a solvate thereof and a pharmaceutically acceptable carrier therefor.

The DL-Tartrate or a solvate thereof is normally administered in unit dosage form.

The active compound may be administered by any suitable route but usually by the oral or parenteral routes. For such use, the compound will normally be employed in the form of a pharmaceutical composition in association with a pharmaceutical carrier, diluent and/or excipient, although the exact form of the composition will naturally depend on the mode of administration.

Compositions are prepared by admixture and are suitably adapted for oral, parenteral or topical administration, and as such may be in the form of tablets, capsules, oral liquid preparations, powders, granules, lozenges, pastilles, reconstitutable powders, injectable and infusable solutions or suspensions, suppositories and transdermal devices. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

Solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the active compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the active compound is suspended in the vehicle instead of being dissolved and sterilised by exposure to ethylene oxide before suspending in the sterile vehicle. Advantageously, a surfactant or wetting agent is included in the composition to facilitate uniform distribution of the active compound.

As is common practice, the compositions will usually be accompanied by written or printed directions for use in the medical treatment concerned.

As used herein the term 'pharmaceutically acceptable' embraces compounds, compositions and ingredients for both human and veterinary use: for example the term 'pharmaceutically acceptable salt' embraces a veterinarily acceptable salt.

The present invention further provides a method for the treatment and/or prophylaxis of diabetes mellitus, conditions associated with diabetes mellitus and certain complications thereof, in a human or non-human mammal which comprises administering an effective, non-toxic, amount of DL-Tartrate or a solvate thereof to a human or non-human mammal in need thereof.

Conveniently, the active ingredient may be administered as a pharmaceutical composition hereinbefore defined, and this forms a particular aspect of the present invention.

In a further aspect the present invention provides the use of DL-Tartrate or a solvate thereof for the manufacture of a medicament for the treatment and/or prophylaxis of diabetes mellitus, conditions associated with diabetes mellitus and certain complications thereof.

In the treatment and/or prophylaxis of diabetes mellitus, conditions associated with diabetes mellitus and certain complications thereof the DL-Tartrate or a solvate thereof may be taken in amounts so as to provide Compound (I) in suitable doses, such as those disclosed in EP 0,306,228, WO94/05659 or WO98/55122.

No adverse toxicological effects are indicated in the above mentioned treatments for the compounds of the invention.

The following examples illustrate the invention but do not limit it in any way.

### EXAMPLES

### Example 1 5-[4-[2-(N-methyl-N-(2-pyridyl)amino)ethoxy]benzyl]thiazolidine-2,4-dione DL tartrate

A mixture of 5-[4-[2-(N-methyl-N-(2-pyridyl)amino)ethoxy]benzyl]thiazolidine-2,4-dione (10 g) and ethanol (300 ml) was stirred and heated to reflux to give a clear solution. A hot solution of DL-tartaric acid (4.2 g) in ethanol (80 ml) (prepared by heating to reflux for 15 minutes) was added. The solution was stirred at reflux until crystallisation was observed and the mixture was then cooled to 21 °C and stirred at this temperature for 2.5 hours. The product was collected by filtration, washed with ethanol (100 ml) and dried under vacuum over phosphorus pentoxide for 4 hours to provide 5-[4-[2-(N-methyl-N-(2-pyridyl)amino)ethoxy] benzyl]thiazolidine-2,4-dione DL-tartrate (14.2 g) as a white crystalline solid.
¹H-NMR (d6-DMSO): consistent with 5-[4-[2-(N-methyl-N-(2-pyridyl)amino)ethoxy]benzyl]thiazolidine-2,4-dione DL-tartrate.

### Example 2 5-[4-[2-(N-Methyl-N-(2-pyridyl)amino)ethosy]benzyl]thiazolidine-2,4-dione DL-Tartrate

A mixture of 5-[4-[2-(N-methyl-N-(2-pyridyl)amino)ethoxylbenzyl] thiazolidine-2,4-dione (3.0 g) and acetone (50 ml) was heated at reflux with stirring for 50 minutes. To this was added a hot clear solution of DL-tartaric acid (1.27 g) in water (6.0 ml). The reaction mixture was heated at reflux for 5 minutes, then cooled to 21°C. The white solid was collected by filtration, washed with acetone (50 ml) then dried under reduced pressure for 1.8 hours at 21°C to afford 5-[4-[2-(N-methyl-N-(2-pyridyl)amino)ethoxy]benzyl]thiazolidine-2,4-dione DL-tartrate (4.0 g) as a white crystalline solid.

### Characterising data recorded for the product of Example 1

The infrared absorption spectrum of a mineral oil dispersion of the product was obtained using a Nicolet 710 FT-IR spectrometer at 2 cm⁻¹ resolution (Figure 1). Data were digitised at 1 cm⁻¹ intervals. Bands were observed at: 3451, 1751, 1696, 1639, 1630, 1610, 1539, 1513, 1461, 1414, 1378, 1352, 1287, 1269, 1234, 1208, 1175, 1155, 1133, 1076, 1058, 1000, 922, 902, 839, 750, 713, 673, 600, 525, 508 cm⁻¹.

The infrared spectrum of the solid product was recorded using Perkin-Elmer Spectrum One FT-IR spectrometer fitted with a universal ATR accessory. Bands were observed at: 3457, 3070, 2785, 1750, 1694, 1639, 1628, 1610, 1543, 1512, 1462, 1414, 1352, 1314, 1287, 1270, 1233, 1208, 1185, 1175, 1153, 1132, 1075, 1057, 1039, 1001, 983, 922, 902, 838, 827, 775, 749, 712, 668 cm⁻¹.

The Raman spectrum of the product (Figure 2) was recorded with the sample in an NMR tube using a Nicolet 960 E.S.P. FT-Raman spectrometer, at 4 cm⁻¹ resolution with excitation from a Nd:V04 laser (1064 nm) with a power output of 400mW. Bands were observed at: 3103, 3046, 2956, 2924, 2901, 2859, 1749, 1712, 1610, 1584, 1545, 1463, 1444, 1384, 1353, 1333, 1316, 1292, 1240, 1208 1185, 1174, 1152, 1112, 1095, 1039, 984, 922, 902, 828, 778, 742, 667, 637, 622, 601, 540, 471, 422, 401, 347, 282, 108 cm⁻¹.

The X-Ray Powder Diffractogram pattern of the product (Figure 3) was recorded using the following acquisition conditions: Tube anode: Cu, Generator tension: 40 kV, Generator current: 40 mA, Start angle: 2.0 °2θ, End angle: 35.0 °2θ, Step size: 0.02 °2θ , Time per step: 2.5 seconds.Characteristic XRPD angles and relative intensities are recorded in Table 1.

**Table 1**

| **Angle** | **Rel. Intensity** |
|---|---|
| **2-Theta °** | **%** |
| 6.4 | 15.3 |
| 8.3 | 1.7 |
| 10.4 | 3.2 |
| 12.4 | 5 |
| 12.7 | 4.9 |
| 13.4 | 27.8 |
| 14.0 | 18.9 |
| 14.4 | 8.3 |
| 15.2 | 8.5 |
| 16.1 | 10.1 |
| 16.6 | 13.2 |
| 17.1 | 23.6 |
| 17.8 | 100 |
| 18.4 | 10 |
| 18.6 | 24.3 |
| 19.6 | 5 |
| 20.0 | 28.8 |
| 20.3 | 12.3 |
| 20.9 | 22.7 |
| 21.8 | 26.2 |
| 22.1 | 41.8 |
| 22.9 | 17.2 |
| 23.5 | 14.3 |
| 23.8 | 34.4 |
| 24.0 | 22.3 |
| 24.4 | 15.4 |
| 24.9 | 39.6 |
| 25.6 | 14.5 |
| 25.9 | 16.8 |
| 26.7 | 24.5 |
| 26.9 | 26.3 |
| 27.7 | 12.2 |
| 27.9 | 10.6 |
| 28.8 | 33.8 |
| 29.1 | 12.7 |
| 30.0 | 11.9 |
| 30.6 | 17.4 |
| 31.5 | 9.3 |
| 32.0 | 7.3 |
| 32.5 | 6.1 |
| 33.4 | 7.4 |
| 34.2 | 8.7 |
| 34.6 | 10 |

The solid-state NMR spectrum of the product (Figure 4) was recorded on a Bruker AMX360 instrument operating at 90.55 MHz: The solid was packed into a 4 mm zirconia MAS rotor fitted with a Kel-F cap and rotor spun at ca.10 kHz. The ¹³C MAS spectrum was acquired by cross-polarisation from Hartmann-Hahn matched protons (CP contact time 3ms, repetition time 15 s) and protons were decoupled during acquisition using a two-pulse phase modulated (TPPM) composite sequence. Chemical shifts were externally referenced to the carboxylate signal of glycine at 176.4 ppm relative to TMS and were observed at:
177.9, 176.7, 174.6, 169.9, 156.3, 147.9, 137.5, 132.5, 126.3, 117.1, 116.1, 112.6, 111.1, 109.8, 107.2, 74.2, 72.3, 65.9, 55.7, 49.5, 40.2, 38.3, 35.1 ppm.

### Properties of the DL-Tartrate, recorded for the product of Example 1

### Solid State Stability of the DL-Tartrate

The solid state stability of the drug substance was determined by storing approximately 1.0 g of the material in a glass bottle at i) 40°C / 75% Relative Humidity (RH), open exposure, for 1 month and b) at 50°C, closed, for 1 month. The material was assayed by HPLC for final content and degradation products in both cases.
a) 40°C / 75% RH: No significant degradation observed (HPLC assay 97 % initial).
b) 50°C: No significant degradation observed (HPLC assay 99% initial).

### Melting Point of the DL-Tartrate

The melting point of the DL-Tartrate was determined according to the method described in the U.S. Pharmacopoeia, USP 23, 1995, <741> "Melting range or temperature, Procedure for Class Ia", using a Buchi 545 melting point instrument.
Melting Point: 191.7°C

### Tₒₙₛₑₜ of the DL-Tartrate

The Tₒₙₛₑₜ of the drug substance was determined by Differential Scanning Calorimetry using a Perkin-Elmer DSC7 apparatus.
Tₒₙₛₑₜ (10°C/minute, closed pan): 202 °C

### Solubility of the DL-Tartrate

The solubility of the material was determined by adding water in aliquots from 1 to 1000ml to approximately 100mg of drug substance until the powder had dissolved. The visual solubility was confirmed by an HPLC assay of a saturated solution.
Solubility: 1mg/ml.

## Claims

1. A compound 5-[4-[2-(N-methyl-N-(2-pyridyl)amino)ethoxy]benzyl]-thiazolidine-2,4-dione, DL-Tartrate salt or a solvate thereof.

2. A compound according to claim 1 in a solid dosage form.

3. A compound according to claim 1 in a form being capable of pharmaceutical processing in a manufacturing process that requires or generates heat, for example milling; for example heat-drying especially fluid-bed drying or a spray drying; for example hot melt processing; for example heat-sterilisation such as autoclaving.

4. A compound according to claim 1 in a form having been processed in a manufacturing process requiring or generating heat, for example in a milled form; for example in heat-dried form, especially a fluid-bed dried form or a spray dried form; for example in a form having being hot melt processed; for example in a form having being heat-sterilised by such as autoclaving.

5. A process for preparing the DL-Tartrate or a solvate thereof, **characterised in that** 5-[4-[2-(N-methyl-N-(2-pyridyl)amino)ethoxy]benzyl]thiazolidine-2,4-dione (Compound (I)) or a salt thereof, is reacted with a source of DL-tartrate ion and thereafter, if required, a solvate of the resulting DL-Tartrate is prepared; and the DL-Tartrate or a solvate thereof is recovered.

6. A pharmaceutical composition comprising 5-[4-[2-(N-methyl-N-(2-pyridyl)amino)ethoxy]benzyl]thiazolidine-2,4-dione DL- tartrate or a solvate thereof, and a pharmaceutically acceptable carrier therefor.

7. A compound 5-[4-[2-(N-methyl-N-(2-pyridyl)amino)ethoxy]benzyl]-thiazolidine-2,4-dione DL- tartrate or a solvate thereof for use as an active therapeutic substance.

8. A use of 5-[4-[2-(N-methyl-N-(2-pyridyl)amino)ethoxy]benzyl]thiazolidine-2,4-dione DL- tartrate or a solvate thereof, for the manufacture of a medicament for the treatment and/or prophylaxis of diabetes mellitus, conditions associated with diabetes mellitus and certain complications of conditions associated with diabetes mellitus including renal disease.

## Patentansprüche

1. 5-[4-[2-(N-Methyl-N-(2-pyridyl)amino)ethoxy]benzyl]-thiazolidin-2,4-dion, ein DL-Tartratsalz oder ein Solvat davon.

2. Verbindung gemäß Anspruch 1 in einer festen Dosierungsform.

3. Verbindung gemäß Anspruch 1 in einer Form, welche pharmazeutisch verarbeitbar ist in einem Herstellungsverfahren, welches Hitze erfordert oder erzeugt, beispielsweise Mahlen; beispielsweise Hitzetrocknung, insbesondere Fließbetttrocknen oder Sprühtrocknen; beispielsweise Heißschmelzverarbeitung; beispielsweise Hitzesterilisation, wie Autoklavieren.

4. Verbindung gemäß Anspruch 1 in einer Form, welche verarbeitet wurde in einem Herstellungsverfahren, welches Hitze erfordert oder erzeugt, beispielsweise in gemahlener Form; beispielsweise in hitzegetrockneter Form, insbesondere in fließbettgetrockneter Form oder sprühgetrockneter Form; beispielsweise in heißschmelzverarbeiteter Form; beispielsweise in hitzesterilisierter, wie beispielsweise autoklavierter Form.

5. Verfahren zur Herstellung des DL-Tartrats oder Solvats davon, **dadurch gekennzeichnet, dass** 5-[4-[2-(N-Methyl-N-(2-pyridyl)amino)ethoxy]benzyl]-thiazolidin-2,4-dion (Verbindung (I)) oder ein Salz davon umgesetzt wird mit einer DL-Tartrat-Ionenquelle, woraufhin, falls erforderlich, ein Solvat des resultierenden DL-Tartrats hergestellt wird; und das DL-Tartrat oder ein Solvat davon gewonnen wird.

6. Arzneimittel, umfassend 5-[4-[2-(N-Methyl-N-(2-pyridyl)amino)ethoxy]benzyl]-thiazolidin-2,4-dion DL-Tartrat oder ein Solvat davon und einen pharmazeutisch verträglichen Träger dafür.

7. 5-[4-[2-(N-Methyl-N-(2-pyridyl)amino)ethoxy]benzyl]-thiazolidin-2,4-dion, ein DL-Tartrat oder ein Solvat davon zur Verwendung als therapeutisch wirksamer Stoff.

8. Verwendung von 5-[4-[2-(N-Methyl-N-(2-pyridyl)amino)ethoxy]benzyl]-thiazolidin-2,4-dion, einem DL-Tartrat oder einem Solvat davon zur Herstellung eines Medikaments zur Behandlung und/oder Vorbeugung von Diabetes Mellitus, mit Diabetes Mellitus in Verbindung stehenden Zuständen und bestimmten Komplikationen bei mit Diabetes Mellitus in Verbindung stehenden Zuständen, einschließlich renaler Erkrankung.

## Revendications

1. Sel DL-tartrate de 5-[4-[2-(N-méthyl-N-(2-pyridyl)amino)éthoxy]benzyl]-thiazolidine-2,4-dione ou un de ses produits de solvatation.

2. Composé suivant la revendication 1, sous une forme posologique solide.

3. Composé suivant la revendication 1, sous une forme apte au traitement pharmaceutique dans un procédé de production qui a besoin ou qui engendre de la chaleur, par exemple un broyage ; par exemple un séchage thermique, notamment un séchage en lit fluide ou un séchage par atomisation, par exemple un traitement en masse fondue à chaud ; par exemple une stérilisation à chaud telle qu'un autoclavage.

4. Composé suivant la revendication 1, sous une forme ayant été traitée dans un procédé de production ayant besoin ou produisant de la chaleur, par exemple sous une forme broyée ; par exemple sous une forme séchée à chaud, notamment sous une forme séchée en lit fluide ou une forme séchée par atomisation ; par exemple sous une forme ayant subi un traitement en masse fondue à chaud ; par exemple sous une forme ayant subi une stérilisation thermique telle qu'un autoclavage.

5. Procédé pour la préparation du DL-tartrate ou d'un de ses produits de solvatation, **caractérisé en ce que** de la 5-[4-[2-(N-méthyl-N-(2-pyridyl)amino)éthoxy]benzyl]-thiazolidine-2,4-dione (composé (I)) ou un de ses sels est amené à réagir avec une source d'ion DL-tartrate, et ensuite, si besoin, un produit de solvatation du DL-tartrate résultant est préparé ; et le DL-tartrate ou un de ses produits de solvatation est recueilli.

6. Composition pharmaceutique comprenant du DL-tartrate de 5-[4-[2-(N-méthyl-N-(2-pyridyl)amino)éthoxy]-benzyl]-thiazolidine-2,4-dione ou un de ses produits de solvatation et un support pharmaceutiquement acceptable à cette fin.

7. Composé DL-tartrate de 5-[4-[2-(N-méthyl-N-(2-pyridyl)amino)éthoxy]benzyl]-thiazolidine-2,4-dione ou un de ses produits de solvatation, destiné à être utilisé comme substance thérapeutique active.

8. Utilisation du DL-tartrate de 5-[4-[2-(N-méthyl-N-(2-pyridyl)amino)éthoxy]benzyl]-thiazolidine-2,4-dione ou d'un de ses produits de solvatation pour la production d'un médicament destiné au traitement et/ou à la prophylaxie du diabète sucré, d'affections associées au diabète sucré, de certaines complications d'affections associées au diabète sucré, y compris une maladie rénale.
